# EUROPEAN PATENT SPECIFICATION

(11) **EP 4 076 579 B1**
(45) Date of publication and mention of the grant of the patent: **11.06.2025**
(21) Application number: 20839275.3
(22) Date of filing: 17.12.2020
(51) Int. Cl.: A61M 5/14, A61M 1/00, A61M 3/02, A61M 5/44, A61M 5/142, A61M 39/24

(54) **PREPARATION AND DELIVERY OF FLUIDS FOR SURGERY**
VORBEREITUNG UND ABGABE VON FLÜSSIGKEITEN FÜR DIE CHIRURGIE
PRÉPARATION ET ADMINISTRATION DE FLUIDES POUR INTERVENTION CHIRURGICALE

(30) Priority: 20.12.2019 GB 201919087
(43) Date of publication of application: 26.10.2022
(73) Proprietor: Gamida Tech, 95600 Eaubonne (FR)
(72) Inventor: HILES, Peter, Datchet Berkshire SL3 9EG (GB); KNOWLES, Stephen, Datchet Berkshire SL3 9EG (GB); HABERMAN, Jessica, Datchet Berkshire SL3 9EG (GB); COLLINGS, Simon, Datchet Berkshire SL3 9EG (GB)
(74) Representative: Serjeants LLP
(86) International application number: PCT/EP2020/086705
(87) International publication number: WO 2021/122947

(56) References cited:
- WO-A1-00/57927
- US-A1- 2009 157 016
- US-A1- 2013 079 702

## Description

### Technical field

The invention relates to the field of surgical procedures, during which a fluid is delivered to a patient and, in most cases, simultaneously drained from the patient. A particular application of the invention is found in hyperthermic intra-peritoneal chemotherapy (HIPEC), during which a heated fluid containing chemotherapy drugs is pumped into the abdominal cavity of a patient. However, the invention has wider applications in the delivery of fluids to a patient during surgical or other medical procedures, especially when the fluids require a preparation step such as heating prior to delivery.

The fluid is typically based on saline. It need not contain a drug or other biologically active ingredient: it could be used purely to maintain fluid pressure or hydration in the patient. Alternatively, the fluid could be a bodily fluid such as blood or plasma that is first removed from the patient, treated and then recirculated back to the patient.

The patient may be human or animal.

### Background of the invention

HIPEC is a procedure that is used following surgery to remove cancerous tumours from inside the abdominal (or peritoneal) cavity. In order to kill cancerous cells that might remain, a heated saline solution containing one or more chemotherapy drugs is pumped into the peritoneal cavity to bathe the abdominal organs for a period of time: normally at least 45 to 90 minutes. By heating the fluid above ambient temperature, e.g. to 42°C, the efficacy of the chemotherapy drugs is increased. Dedicated apparatus is provided for, in a recirculation mode, pre-treating the fluid by using a pump to circulate the fluid through a heated reservoir until it reaches the required temperature; then, in a delivery mode, pumping the pre-treated fluid into the abdominal cavity of the patient.

There are two variants of the HIPEC procedure. In closed HIPEC, at the end of the surgery a delivery tube and a return tube are inserted into the abdominal cavity and the main incision is stitched to close the cavity and substantially seal around those tubes. The HIPEC apparatus then pumps fluid into the cavity through the delivery tube. When the cavity is full, continued pumping causes the excess fluid to return to the apparatus via the return tube, owing to the internal pressure in the cavity. In open HIPEC, at the end of the surgery a delivery tube and a return tube are inserted into the abdominal cavity, which is left open. The HIPEC apparatus then pumps fluid into the cavity through the delivery tube. The apparatus simultaneously pumps excess fluid out of the cavity to return to the apparatus via the return tube. One advantage of open HIPEC is that the surgeon continues to have access to the abdominal cavity during the HIPEC process and can manipulate the abdominal organs to ensure that the fluid reaches all parts of them. A disadvantage is that it requires a second pump to drain the fluid from the patient.

The known HIPEC apparatus requires manual input to switch the apparatus between the recirculation mode and the delivery mode by rotating a dial. It would be preferable for switching to be under automatic control. The apparatus should also be suitable for use in both open and closed HIPEC procedures.

Because the delivery and return tubes must be inserted into the abdominal cavity at the end of the surgical procedure, they need to be kept sterile both inside and outside, while the HIPEC apparatus generally needs to be kept sterile only on the internal surfaces that come into contact with the fluid. There is a need for a convenient and reliable method of connecting the sterile delivery and return tubes to the non-sterile HIPEC apparatus.

Published patent application US 2013/079702 A1 discloses a surgical fluid management system that is not intended for HIPEC but for distending a uterine cavity to allow cutting and extraction of abnormal uterine tissue. The system comprises a fluid source, fluid delivery lines, one or more pumps, and a filter for re-circulating the fluid between the source and the uterine cavity.

Published patent application WO 00/57927 A1 discloses an arrangement for preparing sterile fluid at a predetermined temperature and pressure for medical use: specifically for peritoneal dialysis. The fluid may be heated by passing it through a coil in a bath of heating fluid, while a pump recirculates the heating fluid in the bath to homogenize its temperature. Similarly, The fluid may be cooled by passing it through a coil in a bath of cooling fluid, while a pump recirculates the cooling fluid in the bath to homogenize its temperature.

### Summary of the invention

The invention provides a medical apparatus for delivering fluid to a patient, as defined in claim 1. The scope of the invention is defined by the appended set of claims.

Features of the invention that are preferred but not essential are defined in the dependent claims.

One advantage of the fluid delivery apparatus of the present invention is the ease with which it can be switched from the recirculation mode to the fluid delivery mode, simply by reversing the direction of the first pump. This means that the mode can be changed automatically or under electronic control, without the need to manually turn a valve, for example.

Preferably, when the pump reverses, the valve assembly will respond to the change in pressure at the second valve port by opening one and closing the other of the first and second valves to direct fluid flow in the appropriate manner. However, the invention in its broadest scope does not exclude other means of achieving the same result, such as operating the valves under automatic control or replacing the first and second valves with a single, three-way valve.

### The drawings

Figure 1 is a perspective view of a fluid delivery apparatus according to the invention.
Figure 2 is a perspective view of the removable body of an apparatus according to the invention, including the reservoir and the valve assembly.
Figure 3 is a cut-away perspective view of the valve assembly of Figure 2.
Figure 4 is a vertical section through the filter tray of the apparatus Figure 2, showing a tube connector according to the invention.
Figure 5 is a schematic diagram of the apparatus according to the invention, showing the flow of fluids during a recirculation mode of operation.
Figure 6 is a view similar to Figure 2, showing the flow of fluids during the recirculation mode of operation.
Figure 7 is a schematic diagram of the apparatus according to the invention, showing the flow of fluids during a delivery mode of operation in a closed HIPEC procedure.
Figure 8 is a view similar to Figure 2, showing the flow of fluids during a delivery mode of operation in the closed HIPEC procedure.
Figure 9 is a schematic diagram of the apparatus according to the invention, showing the flow of fluids during a delivery mode of operation in an open HIPEC procedure.
Figure 10 is a view similar to Figure 2, showing the flow of fluids during a delivery mode of operation in the open HIPEC procedure.

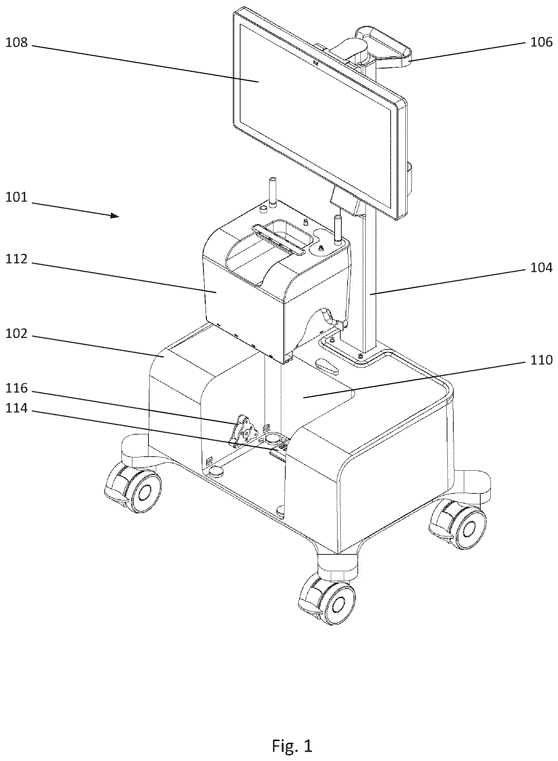

Figure 1 shows medical apparatus 101 for delivering fluid to a patient during a HIPEC procedure, in accordance with the invention. The apparatus 101 comprises a wheeled trolley 102, on which the apparatus can be moved around. A column 104 carries a handle 106 for steering the trolley 102, and a screen 108, which can display information to an operator and may also be an interactive touch screen through which the operator can input instructions to the apparatus. The centre of the trolley 102 contains a recess 110, into which a removable body 112 of the apparatus can be lowered. The removable body 112 will be described in more detail below. It is intended to be removed and replaced after each use of the apparatus to deliver fluid to a patient during a surgical procedure, while the other parts of the apparatus 101 can be re-used in multiple procedures. The recess 110 contains electrical contacts 114 for providing power and control signals to the removable body 112. Projecting from each side wall of the recess 110 is the rotor 116 of a peristaltic pump.

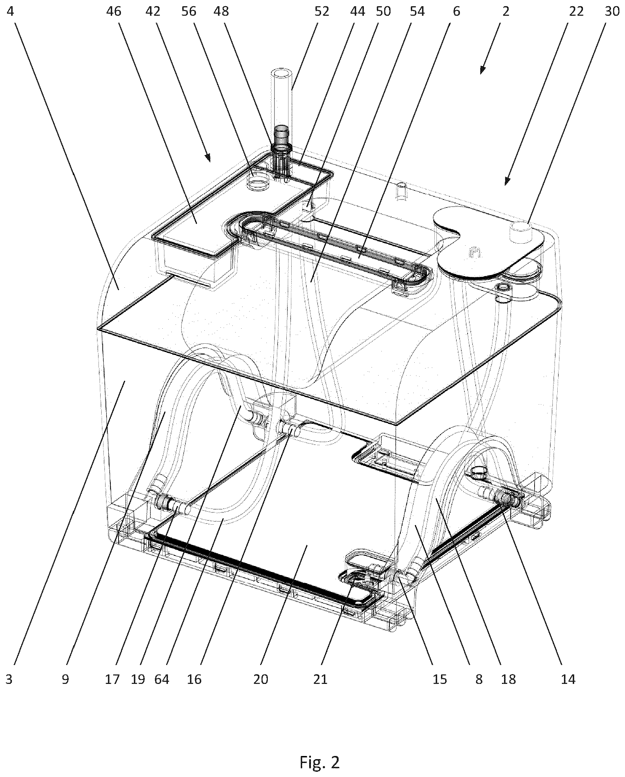

Figure 2 is a perspective view of the removable body 112 of Figure 1. It principally comprises a reservoir 2, which is formed by securing together a lower part 3 that can contain a fluid and an upper part 4 that acts as a cover. The upper part 4 includes a handle 6 to facilitate lifting the removable body 112 into and out of the recess 110 of the trolley 102.

A first side wall of the reservoir 2 is moulded with an arcuate recess that forms a track 8 for a first peristaltic pump 10. Near the ends of the arcuate recess, the first side wall is penetrated by first and second pump ports 14,15. The opposite, second side wall of the reservoir 2 is similarly moulded with an arcuate recess that forms a track 9 for a second peristaltic pump 12, and is penetrated by third and fourth pump ports 16,17. The first and second pump ports 14,15 are connected to one another by a first compressible tube 18 that runs around the track 8 of the first peristaltic pump 10. In a well known manner, the first peristaltic pump 10 comprises a hub that is mounted for rotation about a horizontal axis and supports a set of rollers about its circumference. The rollers in one sector of the circumference compress the tube against the track 8, trapping portions of fluid in the uncompressed lengths of tube between them. By rotating the hub, the rollers are caused to move around the track and force the portions of fluid to move with them. The direction of the first pump 10 is reversible so that it can be operated to pump fluid from the first pump port 14 to the second pump port 15 or vice versa. The second peristaltic pump 12 is configured in an identical manner to pump fluid between the third and fourth pump ports 16,17 via a second compressible tube 19, although for the purpose of the present invention it is not essential that the second pump 12 should be reversible.

A heating panel 20 is provided in the base of the reservoir 2. A temperature probe 21 is provided within the reservoir 2 and adjacent to the second pump port 15 to measure the temperature of fluid as it emerges from the second pump port 15.

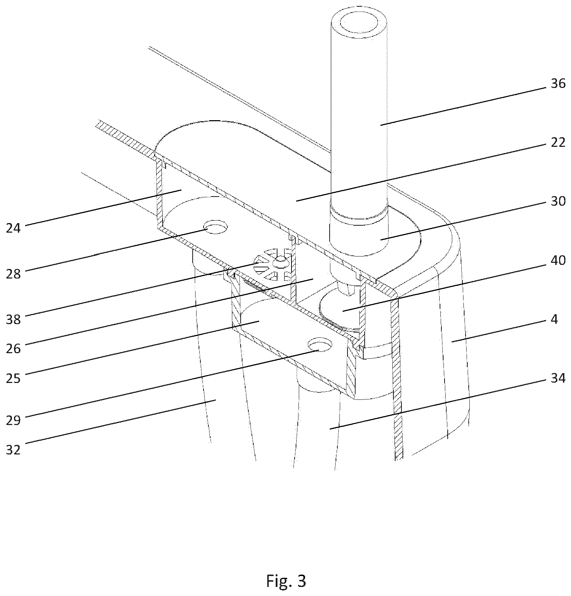

A valve assembly 22 is supported by the cover 4 of the reservoir 2. Details of the valve assembly 22 are shown in the cut-away view of Figure 3. The valve assembly 22 comprises first, second and third chambers 24,25,26 associated respectively with first, second and third valve ports 28,29,30.

The first valve port 28 opens between the first valve chamber 24 and the interior of the reservoir 2. It is coupled to the upper end of an intake tube 32, the lower end of which opens into the reservoir 2 below the expected fluid level.

The second valve port 29 opens between the second valve chamber 25 and the interior of the reservoir 2. It is coupled to the upper end of a first transfer tube 34, the lower end of which is coupled to the first pump port 14, whereby the first transfer tube 34 passes through the interior of the reservoir 2 but fluid contained within the first transfer tube 34 remains separate from the bulk fluid in the reservoir 2.

The third valve port 30 opens between the third valve chamber 26 and the exterior of the reservoir 2. During use of the apparatus it is coupled to the proximal end of a delivery tube 36 for delivery of fluid to a patient, as described below.

The first and second valve chambers 24,25 are connected via a first valve 38, which permits fluid to flow from the first valve chamber 24 to the second valve chamber 25 but not in the opposite direction. The second and third valve chambers 25,26 are connected via a second valve 40, which permits fluid to flow from the second valve chamber 25 to the third valve chamber 26 but not in the opposite direction. There is no direct connection between the first and third valve chambers 24,26. In the preferred embodiment of the invention the valves 38,40 are umbrella valves but other types of one-way valve would be acceptable alternatives.

A filter assembly 42 is also supported by the cover 4 of the reservoir 2. The filter assembly 42 comprises a tray that is divided into a suction chamber 44 and a filter chamber 46. There is no direct communication between the suction chamber 44 and the filter chamber 46 so they could alternatively be formed as separate components.

The suction chamber 44 is airtight, except for a suction return port 48 and a suction outlet port 50. The suction return port 48 opens to the exterior of the reservoir 2. During certain uses of the apparatus it may be coupled to the proximal end of a suction return tube 52 for receiving fluid from a patient, as described below. The suction outlet port 50 opens to the interior of the reservoir 2. It is coupled to the upper end of a second transfer tube 54, the lower end of which is coupled to the third pump port 16, whereby the second transfer tube 54 passes through the interior of the reservoir 2 but fluid contained within the second transfer tube 54 remains separate from the bulk fluid in the reservoir 2.

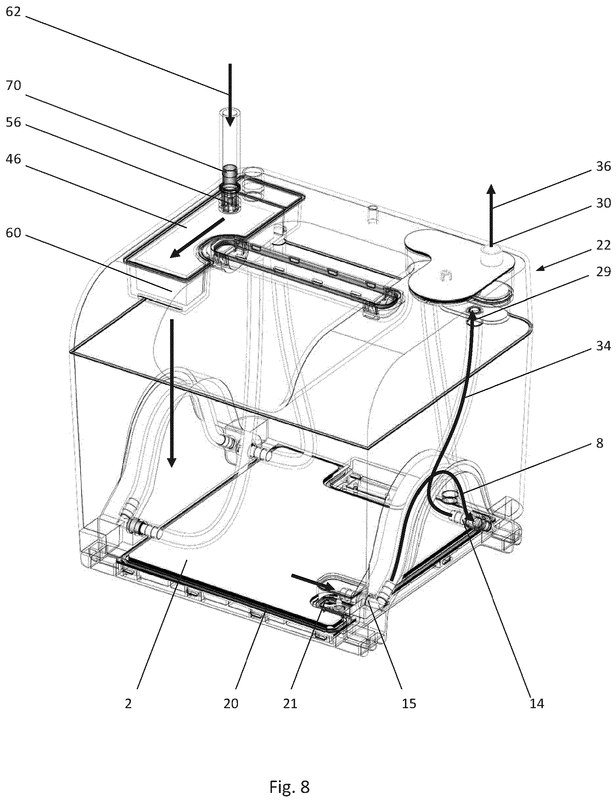

The filter chamber 46 comprises a gravity return port 56, a filter inlet port 58 (hidden from view in Figure 2) and a filter outlet port 60 (also hidden from view in Figure 2). The gravity return port 56 opens to the exterior of the reservoir 2. During certain uses of the apparatus it may be coupled to the proximal end of a gravity return tube 62 (Figure 8) for receiving fluid from a patient, as described below. The filter inlet port 58 opens to the interior of the reservoir 2. It is coupled to the upper end of a third transfer tube 64, the lower end of which is coupled to the fourth pump port 17, whereby the third transfer tube 64 passes through the interior of the reservoir 2 but fluid contained within the third transfer tube 64 remains separate from the bulk fluid in the reservoir 2. The filter outlet port 60 also opens to the interior of the reservoir 2, such that fluid may fall into the reservoir from the filter outlet port 60. A tube (not shown) may be coupled to the filter outlet port 60 to guide the falling fluid and prevent splashing. The filter chamber 46 also contains a filter 66 (not shown in Figure 2), arranged such that fluid entering the chamber 46 via the gravity return port 56 or the filter inlet port 58 has to pass through the filter 66 before it can exit the chamber 46 via the filter outlet port 60. The filter 66 may be formed of any suitable porous or open-mesh material, certified for medical use, which is capable of trapping and/or absorbing particulate matter or adsorbing chemical or biological constituents of the fluid returning from the abdominal cavity of a patient, which it is desired to prevent from being recirculated to the patient again.

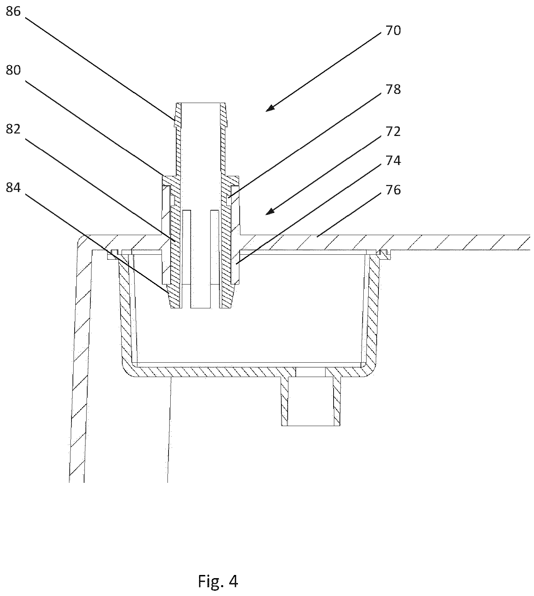

Figure 4 illustrates a connector 70 which is useful for coupling an external tube to an exterior port 72 of a medical apparatus for delivering fluid to a patient or receiving fluid from a patient. For example, in the illustrated fluid delivery apparatus, the connector 70 may be used to couple the delivery tube 36 to the third valve port 30; the suction return tube 52 to the suction return port 48; or the gravity return tube 62 to the gravity return port 56. Each connector 70 may be identical so that it can engage with any port 72. However, it is preferred that each connector 70 and its corresponding port 72 are provided with complementary features such as keyways (not shown) that prevent the wrong connector 70 being coupled to the wrong port 72. The connectors 70 and ports 72 may be labelled, e.g. by colour coding, to make the correspondence apparent to users.

Ignoring the presence of any keyways, as just discussed, the port 72 consists of a simple, cylindrical collar 74 that extends through a wall 76 of the apparatus and is open at both ends. The connector 70 is essentially a smaller cylinder that fits snugly through the collar 74 of the port 72. An O-ring 78 is seated in an outward-facing circumferential channel of the connector 70 to seal between the connector 70 and the port 72. A flange 80 projects radially from the connector 70 so that when the connector 70 is inserted into the port 72, the flange 80 butts against the outer end of the collar 74 and prevents the connector 70 being inserted further. The proximal end of the connector 70 is divided by slots into a number of longitudinal arms 82. In the illustrated embodiment, the number of arms 82 is four. Each of the arms 82 ends in an outwardly-projecting tang 84, which has a radius greater than the internal radius of the collar 74 such that, as the connector 70 is inserted into the port 70, the tangs 84 force the arms to bend inwards to fit through the collar 74. Preferably the outer surfaces of the tangs 84 are tapered to facilitate inserting them into the collar 74. Just before the flange 80 comes into contact with the distal end of the collar 74, the tangs pass the proximal end of the collar 74, thereby allowing the resilient arms 82 to spring outwards and the tangs 84 to form a snap-fit engagement with the proximal end of the collar 74, as seen in Figure 4. This prevents the connector 70 being withdrawn from the collar 74. The distal end of the connector 70 comprises a circumferential barb 86 or a series of ridges (not shown) that permit the proximal end of a flexible tube (not shown) to be pushed onto the connector and retained by the barb 86 so that it cannot easily be removed.

Because the distal end of the flexible tube is generally implanted into the patient during surgery, the tube and its attached connector 70 need to be kept sterile, while the external surfaces of the fluid delivery apparatus are typically not sterile. For this reason, the connector 70 is first attached to the tube and remains with it during sterilization and surgery. When the fluid delivery apparatus is needed, it is brought into the non-sterile area of the operating theatre and the tube is then connected to it by inserting the connector 70 into the port 72 as just described. When doing that, the operator grips the outside of the tube in the region of the barb 86 and there is no need for them to touch the port 72 or any other part of the unsterilized apparatus. Until the connectors 70 are attached, the external ports of the apparatus are preferably closed by removable caps (not shown) to prevent contamination of the interior of the apparatus.

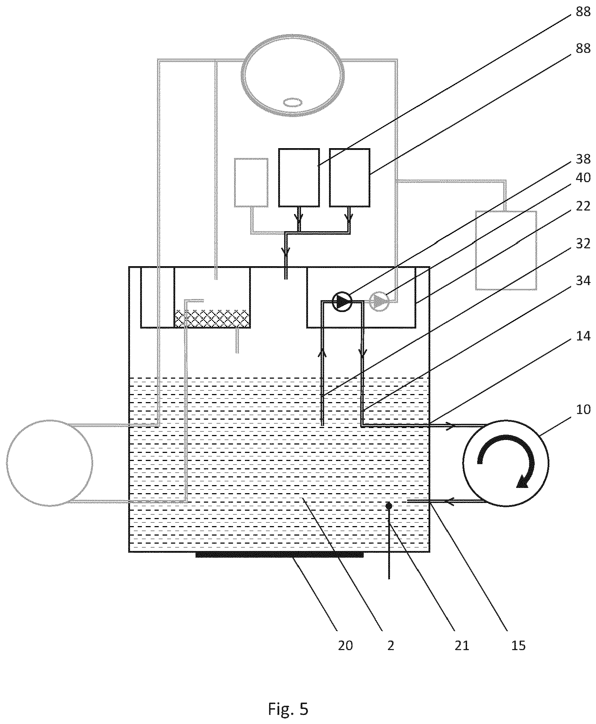

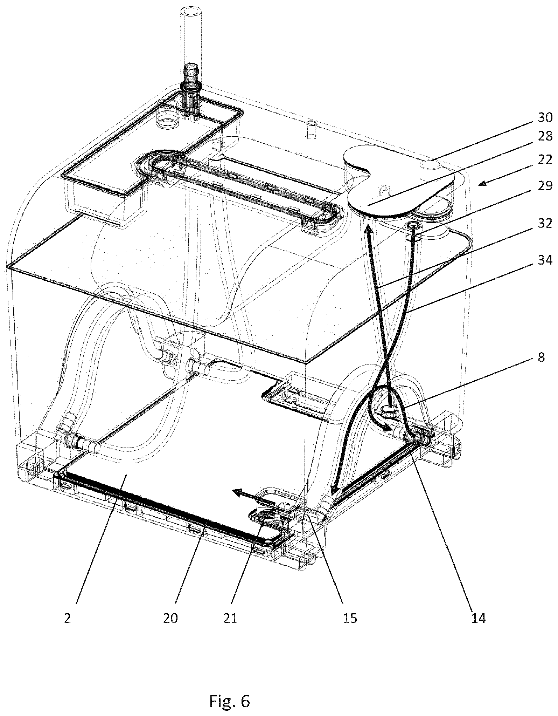

Figures 5 and 6 illustrate the flow of fluids through the fluid delivery apparatus during an initial, recirculation mode of operation. Figure 6 marks the flow path on a copy of Figure 2 and the same features will not be described again here. Figure 5 shows the flow path in schematic form (not to scale). Fluid conduits are shown as open lines, with V-shaped arrows to indicate the direction of flow. Parts of the apparatus that are active in this mode are shown in black lines, while those not involved in this mode are shown in grey.

The recirculation mode of operation is used to prepare the fluid for delivery to the patient, in particular by pre-heating it to the desired temperature for the HIPEC procedure. A suitable volume of saline fluid is first delivered from a supply 88 into the reservoir 2 and the heater 20 is switched on to warm the fluid at the base of reservoir. Although the heat will spread through the fluid by conduction and convection, it can be brought to a more uniform temperature by using the first pump 10 to recirculate the fluid. The pump 10 is operated in a first direction (shown as clockwise in Figure 5) that pumps fluid from the first pump port 14 to the second pump port 15. This lowers the pressure at the second valve port 29 and draws fluid from the reservoir 2 via the valve assembly 22. The path followed by the recirculating fluid is thus: from the reservoir 2 through intake tube 32 into the first valve port 28; through the first valve 38 to exit the valve assembly 22 via the second valve port 29; through the first transfer tube 34 to the first pump port 14 and around the first pump 10 to return to the reservoir 2 via the second pump port 15. Because of the reduced pressure at the second valve port 29, the second valve 40 remains closed.

The temperature probe 21 monitors the temperature of the fluid as it emerges from the second pump port 15 and sends measurements to a controller (not shown) that can display the information to a human operator and/or use the information for automatic control of the heater 20 and the first pump 10. The controller may also be used for manual and/or automatic control of the second pump 12, the supplies of saline 88 and chemotherapy drugs 89 and any other aspects of the apparatus. For this purpose it may receive signals from other sensors (not shown), e.g. to monitor the temperature of the fluid at different locations, or the condition of the fluid returning from a patient.

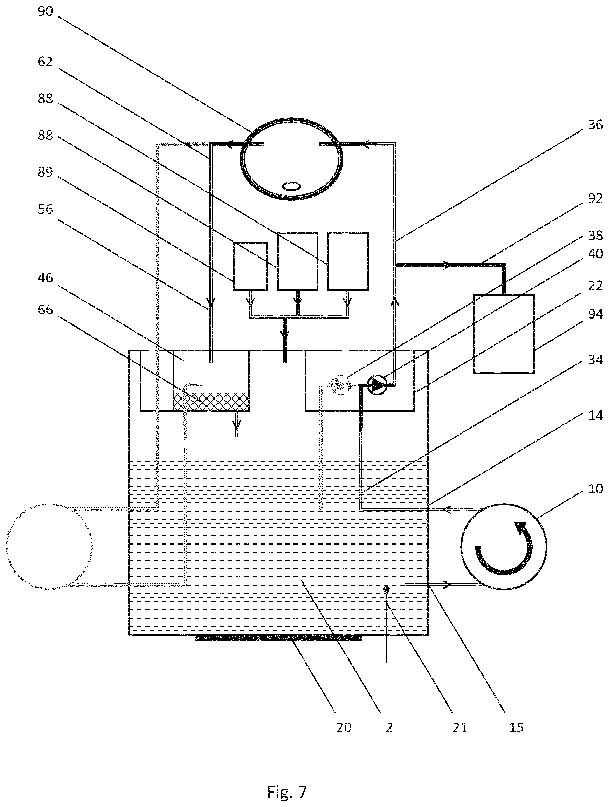

Figures 7 and 8 are similar to Figures 5 and 6, respectively, but illustrate the flow of fluids through the fluid delivery apparatus during a delivery mode of operation in a closed HIPEC procedure. As previously explained, in closed HIPEC heated saline containing chemotherapy drugs is pumped into the body cavity 90 of the patient. The cavity 90 being closed, pressure in the cavity 90 causes fluid to be discharged via a return tube 52 and return - normally assisted by gravity - to the fluid delivery apparatus.

In this mode, the first pump 10 is operated in the direction (shown as anti-clockwise in Figure 7) opposite to that in the recirculation mode. This draws fluid from the reservoir 2 via the second pump port 15 and pumps it to the first pump port 14, from where it travels along the first transfer tube 34 and increases the pressure at the second valve port 29. This closes the first valve 38 and opens the second valve 40 to allow fluid to emerge from the valve assembly 22 via the third valve port 30. From there, it passes along the delivery tube 36 to the body cavity 90 of the patient. Fluid discharged from the body cavity 90 flows along the gravity return tube 62 to enter the filter chamber 46 through the gravity return port 56. The fluid must pass through the material of the filter 66, which traps particles and optionally other contaminants, before the filtered fluid flows out of the filter outlet port 60 and falls back into the reservoir 2.

At some stage, the supply 89 of chemotherapy drugs is turned on to add those drugs to the fluid in the reservoir, from where the saline fluid can carry them to the patient. The apparatus is capable of introducing the chemotherapy drugs after the circulation shown in Figures 7 and 8 has already been established with pure saline or, according to medical need, the drug supply 89 may be opened earlier, e.g. during the recirculation mode of operation, to ensure good mixing of the drugs with the saline before delivery to the patient. Similarly, a fixed dose of the chemotherapy drugs may be delivered at the outset or the supply my be continuous or intermittent as the need arises.

The apparatus is further provided with a waste tank 94 coupled to the delivery tube 36 by a discharge tube 92. A valve (not shown) may be operated by the controller to divert circulating fluid into the waste tank 94, for example in the event that there is excess fluid in the system or the fluid needs to be refreshed with clean fluid from the supply 88.

The heater 20 may continue to operate during the delivery mode of the apparatus, in order to replace heat lost by the fluid as it circulates via the patient. It may be operated at selective times or at a power level determined in response to signals from the temperature sensor 21, which can monitor the temperature of the fluid entering the second pump port 15, or signals from other temperature sensors (not shown), e.g. for monitoring the temperature of fluid as it passes through the body cavity of the patient or when it returns to the reservoir.

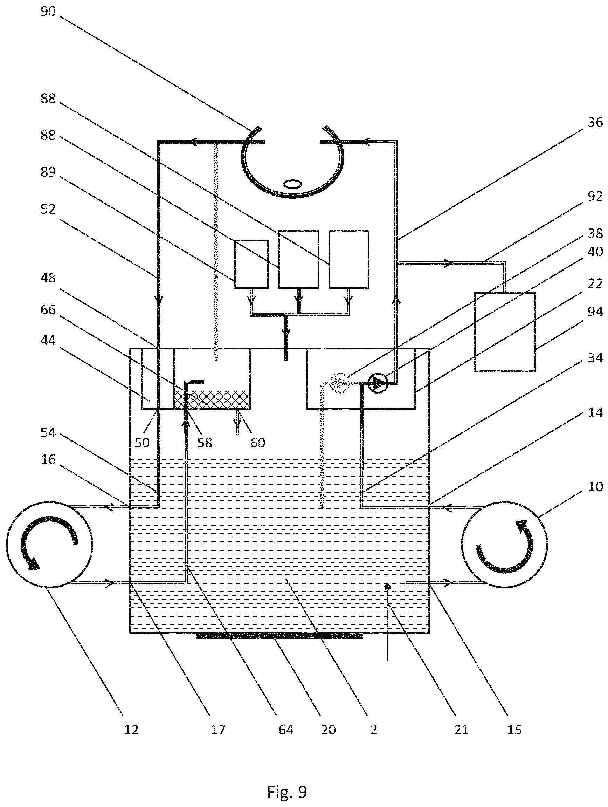

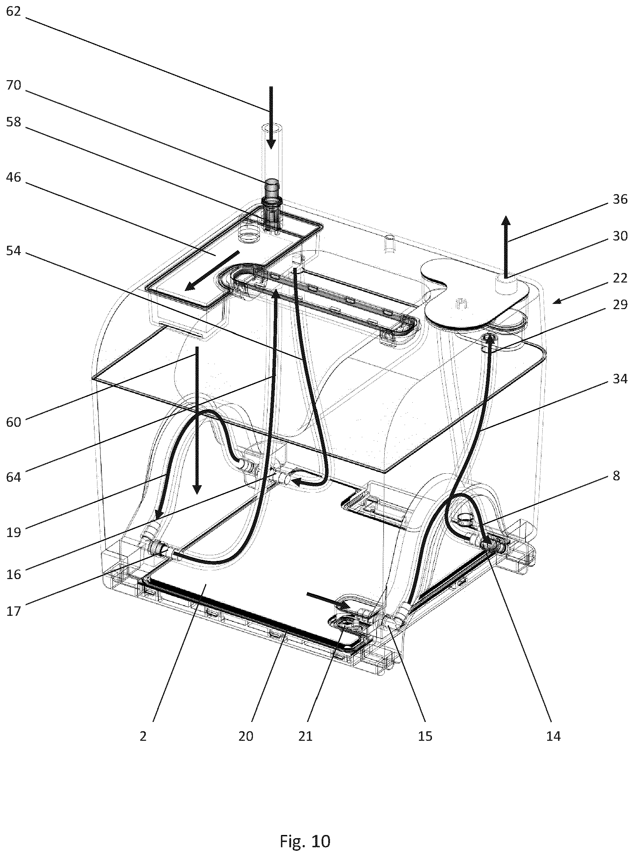

Figures 9 and 10 are similar to Figures 7 and 8, respectively, but illustrate the flow of fluids through the fluid delivery apparatus during a delivery mode of operation in an open HIPEC procedure. As previously explained, in open HIPEC heated saline containing chemotherapy drugs is pumped into the body cavity 90 of the patient. The cavity 90 being open, it remains at ambient pressure so fluid to be discharged needs to be pumped away. For this purpose the second pump 12 is brought into operation.

The first pump 10 and the valve assembly 22 operate in just the same manner as with closed HIPEC, described above with reference to Figures 7 and 8. However, when set up for open HIPEC, the return tube is connected as a suction return tube 52 to the suction return port 48 instead of the gravity return port 56. As previously described, this might require the use of a different connector 70 on the return tube 52 if the ports 48,56 and connectors 70 have complementary keyways to prevent them being wrongly paired up.

The second pump 12 is operated in a direction (shown as anti-clockwise in Figure 9) that pumps fluid from the third pump port 16 to the fourth pump port 17. This creates low pressure in the suction chamber 44 and draws fluid from the patient, via the suction return tube 52, into the suction chamber 44. From there, the fluid is drawn out via the suction outlet port 50 and the second transfer tube 54 to pass through the second pump 12 along the second compressible tube 19. The fluid is pumped out of the fourth pump port 17 and passes through the third transfer tube 64 to enter the filter chamber 46 through the filter inlet port 58. It must pass through the material of the filter 66, which traps particles and optionally other contaminants, before the filtered fluid flows out of the filter outlet port 60 and falls back into the reservoir 2.

The reader will understand that it is not essential for the fluid paths to be exactly as described in relation to the illustrated embodiment of the invention. For example, the first, second and third transfer tubes pass through the interior of the reservoir 2 only because they need to connect respectively to the first, third and fourth pump ports 14,16,17. Although this keeps the tubes neatly contained within the apparatus, a different physical arrangement could be without changing the essential features of the fluid circuit.

It is not excluded that the apparatus could be set up and operated as described in relation to Figures 9 and 10, even in the case of closed HIPEC. For example, the operation of the second pump 12 to discharge fluid from the patient might be considered beneficial to reduce the pressure in the abdominal cavity 90.

## Claims

1. Medical apparatus for delivering fluid to a patient, comprising:
a reservoir (2) for a fluid;
a first pump (10) comprising first and second pump ports (14,15); and
a valve assembly (22) comprising first, second and third valve ports (28,29,30); wherein:
the first valve port (28) is in fluid communication with the reservoir (2);
the second valve port (29) is in fluid communication with the first pump port (14);
the third valve port (30) is an outlet for delivering fluid to a patient;
the second pump port (15) is in fluid communication with the reservoir (2); and
**characterized in that**
the first pump (10) is reversible such that:
in a recirculation mode, the valve assembly (22) is configured to permit fluid flow from the first valve port (28) to the second valve port (29) but not to the third valve port (30), the pump (10) being operated in one direction to draw fluid from the reservoir (2) through the valve assembly (22) to the first pump port (14) and to recirculate the fluid from the second pump port (15) to the reservoir (2); and
in a fluid delivery mode, the valve assembly (22) is configured to permit fluid flow from the second valve port (29) to the third valve port (30) but not to the first valve port (28), the pump (10) being operated in an opposite direction to draw fluid from the reservoir (2) to the second pump port (15) and to deliver the fluid from the first pump port (14) through the valve assembly (22) to the third valve port (30).

2. Fluid delivery apparatus according to claim 1, wherein the valve assembly (22) is configured such that the only fluid flows permitted between the first, second and third valve ports (28,29,30) are from the first valve port (28) to the second valve port (29) and from the second valve port (29) to the third valve port (30).

3. Fluid delivery apparatus according to claim 1, wherein the valve assembly (22) comprises a first valve (38) permitting one-way flow from the first valve port (28) to the second valve port (29); a second valve (40) permitting one-way flow from the second valve port (29) to the third valve port (30); and no direct communication between the first and third valve ports (28,30).

4. Fluid delivery apparatus according to any preceding claim, wherein the first pump (10) is a peristaltic pump.

5. Fluid delivery apparatus according to claim 4, wherein the peristaltic pump (10) comprises rollers that compress a resilient tube (18) against an arcuate surface (8), the arcuate surface (8) being formed integrally with a wall of the reservoir (2).

6. Fluid delivery apparatus according to any preceding claim, wherein the first and second pump ports (14,15) open into an interior of the reservoir (2), further comprising a first transfer tube (34) that passes through the reservoir (2) to provide the fluid communication between the second valve port (29) and the first pump port (14).

7. Fluid delivery apparatus according to any preceding claim, further comprising:
a heater (20) for heating the fluid in the reservoir (2); and
a temperature sensor (21) arranged to monitor the temperature of fluid in the apparatus and to provide a temperature signal that can be used to control the heater (20).

8. Fluid delivery apparatus according to any of claims 1 to 7, further comprising a gravity return port (56) in fluid communication with a filter (66), whereby fluid introduced through the gravity return port (56) must pass through the filter (66) to drain into the reservoir (2).

9. Fluid delivery apparatus according to any of claims 1 to 7, further comprising:
a suction return port (48);
a filter (66);
a filter inlet port (58) in fluid communication with the filter (66); and
a second pump (12) comprising third and fourth pump ports (16,17); wherein:
the suction return port (48) is in fluid communication with the third pump port (16);
the fourth pump port (17) is in fluid communication with the filter inlet port (58); and
in the fluid delivery mode the second pump (12) is operated to draw fluid from the suction return port (48) to the third pump port (16) and to deliver fluid from the fourth pump port (17) to the filter inlet port (58), whereby fluid introduced through the filter inlet port (58) must pass through the filter (66) to drain into the reservoir (2).

10. Fluid delivery apparatus according to claim 9, wherein the second pump (12) is a peristaltic pump.

11. Fluid delivery apparatus according to claim 10, wherein the peristaltic pump (12) comprises rollers that compress a resilient tube against an arcuate surface, the arcuate surface being formed integrally with a wall of the reservoir (2).

12. Fluid delivery apparatus according to any of claims 9 to 11, wherein the third and fourth pump ports (16,17) are located in the reservoir (2), further comprising a second transfer tube (54) that passes through the reservoir (2) to provide the fluid communication between the suction return port (48) and the third pump port (16).

13. Fluid delivery apparatus according to any of claims 9 to 12, further comprising a gravity return port (56) in direct fluid communication with the filter (66), whereby fluid introduced through the gravity return port (56) must pass through the filter (66) to drain into the reservoir (2).

14. Fluid delivery apparatus according to any preceding claim, further comprising a connector (70) that comprises:
a through bore extending from a first end of the connector (70) to a second end of the connector (70);
a first coupling (86) at the first end for attaching the connector (70) to a flexible tube (36); and
a second coupling at the second end for non-removably attaching the connector (70) to the third valve port (30).

15. Fluid delivery apparatus according to claim 14, wherein the second coupling is a snap-fit coupling, which comprises a plurality of longitudinal resilient arms (82) that pass through the third valve port (30), each arm terminating in a tang (84) that springs radially outwards to engage an end surface of the port (30) and prevent withdrawal of the connector (70) through the port (30).

## Patentansprüche

1. Medizinische Einrichtung zum Abgeben von Fluid an einen Patienten, umfassend:
ein Reservoir (2) für ein Fluid;
eine erste Pumpe (10), umfassend einen ersten und einen zweiten Pumpenanschluss (14, 15); und
eine Ventilanordnung (22), umfassend einen ersten, einen zweiten und einen dritten Ventilanschluss (28, 29, 30); wobei:
der erste Ventilanschluss (28) mit dem Reservoir (2) in Fluidverbindung steht;
der zweite Ventilanschluss (29) mit dem ersten Pumpenanschluss (14) in Fluidverbindung steht;
der dritte Ventilanschluss (30) ein Auslass zum Abgeben von Fluid an einen Patienten ist;
der zweite Pumpenanschluss (15) mit dem Reservoir (2) in Fluidverbindung steht; und **dadurch gekennzeichnet, dass**
die erste Pumpe (10) reversibel ist, so dass:
in einem Rückführungsmodus die Ventilanordnung (22) so konfiguriert ist, dass sie einen Fluidstrom vom ersten Ventilanschluss (28) zum zweiten Ventilanschluss (29), jedoch nicht zum dritten Ventilanschluss (30) zulässt, wobei die Pumpe (10) in eine Richtung betrieben wird, um Fluid aus dem Reservoir (2) durch die Ventilanordnung (22) zum ersten Pumpenanschluss (14) zu ziehen und das Fluid vom zweiten Pumpenanschluss (15) zum Reservoir (2) zurückzuführen; und
in einem Fluidabgabemodus die Ventilanordnung (22) so konfiguriert ist, dass sie einen Fluidstrom vom zweiten Ventilanschluss (29) zum dritten Ventilanschluss (30), jedoch nicht zum ersten Ventilanschluss (28) zulässt, wobei die Pumpe (10) in eine entgegengesetzte Richtung betrieben wird, um Fluid aus dem Reservoir (2) zum zweiten Pumpenanschluss (15) zu ziehen und das Fluid vom ersten Pumpenanschluss (14) durch die Ventilanordnung (22) an den dritten Ventilanschluss (30) abzugeben.

2. Fluidabgabeeinrichtung nach Anspruch 1, wobei die Ventilanordnung (22) so konfiguriert ist, dass die einzigen Fluidströme, die zwischen dem ersten, dem zweiten und dem dritten Ventilanschluss (28, 29, 30) zulässig sind, diejenigen vom ersten Ventilanschluss (28) zum zweiten Ventilanschluss (29) und vom zweiten Ventilanschluss (29) zum dritten Ventilanschluss (30) sind.

3. Fluidabgabeeinrichtung nach Anspruch 1, wobei die Ventilanordnung (22) ein erstes Ventil (38) umfasst, das einen Einwegstrom vom ersten Ventilanschluss (28) zum zweiten Ventilanschluss (29) zulässt; ein zweites Ventil (40) umfasst, das einen Einwegstrom vom zweiten Ventilanschluss (29) zum dritten Ventilanschluss (30) zulässt; und keine direkte Verbindung zwischen dem ersten und dem dritten Ventilanschluss (28, 30) zulässt.

4. Fluidabgabeeinrichtung nach einem der vorstehenden Ansprüche, wobei die erste Pumpe (10) eine Schlauchpumpe ist.

5. Fluidabgabeeinrichtung nach Anspruch 4, wobei die Schlauchpumpe (10) Rollen umfasst, die ein elastisches Rohr (18) gegen eine bogenförmige Oberfläche (8) drücken, wobei die bogenförmige Oberfläche (8) integral mit einer Wand des Reservoirs (2) ausgebildet ist.

6. Fluidabgabeeinrichtung nach einem der vorstehenden Ansprüche, wobei der erste und der zweite Pumpenanschluss (14, 15) in ein Inneres des Reservoirs (2) münden, und weiter umfassend ein erstes Übertragungsrohr (34), das durch das Reservoir (2) hindurchtritt, um die Fluidverbindung zwischen dem zweiten Ventilanschluss (29) und dem ersten Pumpenanschluss (14) bereitzustellen.

7. Fluidabgabeeinrichtung nach einem der vorstehenden Ansprüche, weiter umfassend:
eine Heizung (20) zum Erhitzen des Fluids in dem Reservoir (2); und
einen Temperatursensor (21), der dazu angeordnet ist, die Temperatur von Fluid in der Einrichtung zu überwachen und um ein Temperatursignal bereitzustellen, das zum Steuern der Heizung (20) verwendet werden kann.

8. Fluidabgabeeinrichtung nach einem der Ansprüche 1 bis 7, weiter umfassend einen Schwerkraftrücklaufanschluss (56) in Fluidverbindung mit einem Filter (66), wobei durch den Schwerkraftrücklaufanschluss (56) eingeführtes Fluid durch den Filter (66) hindurchtreten muss, um in das Reservoir (2) abzufließen.

9. Fluidabgabeeinrichtung nach einem der Ansprüche 1 bis 7, weiter umfassend:
einen Saugrücklaufanschluss (48);
einen Filter (66);
einen Filtereinlassanschluss (58) in Fluidverbindung mit dem Filter (66); und
eine zweite Pumpe (12), umfassend einen dritten und einen vierten Pumpenanschluss (16, 17);
wobei:
der Saugrücklaufanschluss (48) mit dem dritten Pumpenanschluss (16) in Fluidverbindung steht;
der vierte Pumpenanschluss (17) mit dem Filtereinlassanschluss (58) in Fluidverbindung steht; und
im Fluidabgabemodus die zweite Pumpe (12) betrieben wird, um Fluid vom Saugrücklaufanschluss (48) zum dritten Pumpenanschluss (16) zu ziehen und um Fluid vom vierten Pumpenanschluss (17) an den Filtereinlassanschluss (58) abzugeben, wobei durch die Filtereinlassanschluss (58) eingeführtes Fluid durch den Filter (66) hindurchtreten muss, um in das Reservoir (2) abzufließen.

10. Fluidabgabeeinrichtung nach Anspruch 9, wobei die zweite Pumpe (12) eine Schlauchpumpe ist.

11. Fluidabgabeeinrichtung nach Anspruch 10, wobei die Schlauchpumpe (12) Rollen umfasst, die ein elastisches Rohr gegen eine bogenförmige Oberfläche drücken, wobei die bogenförmige Oberfläche integral mit einer Wand des Reservoirs (2) ausgebildet ist.

12. Fluidabgabeeinrichtung nach einem der Ansprüche 9 bis 11, wobei sich der dritte und der vierte Pumpenanschluss (16, 17) im Reservoir (2) befinden, und weiter umfassend ein zweites Übertragungsrohr (54), das durch das Reservoir (2) hindurchtritt, um die Fluidverbindung zwischen dem Saugrücklaufanschluss (48) und dem dritten Pumpenanschluss (16) bereitzustellen.

13. Fluidabgabeeinrichtung nach einem der Ansprüche 9 bis 12, weiter umfassend einen Schwerkraftrücklaufanschluss (56) in direkter Fluidverbindung mit dem Filter (66), wobei durch den Schwerkraftrücklaufanschluss (56) eingeführtes Fluid durch den Filter (66) hindurchtreten muss, um in das Reservoir (2) abzufließen.

14. Fluidabgabeeinrichtung nach einem der vorstehenden Ansprüche, weiter umfassend einen Verbinder (70), der umfasst:
eine Durchgangsbohrung, die sich von einem ersten Ende des Verbinders (70) zu einem zweiten Ende des Verbinders (70) erstreckt;
eine erste Kupplung (86) am ersten Ende zum Befestigen des Verbinders (70) an einem flexiblen Rohr (36); und
eine zweite Kupplung am zweiten Ende zum nicht lösbaren Befestigen des Verbinders (70) am dritten Ventilanschluss (30).

15. Fluidabgabeeinrichtung nach Anspruch 14, wobei die zweite Kupplung eine Schnappkupplung ist, die eine Vielzahl von länglichen, elastischen Armen (82) umfasst, die durch den dritten Ventilanschluss (30) hindurchtreten, wobei jeder Arm in einer Angel (84) endet, die radial nach außen federt, um in eine Endfläche des Anschlusses (30) einzugreifen und ein Herausziehen des Verbinders (70) durch den Anschluss (30) zu verhindern.

## Revendications

1. Appareil médical pour administrer un fluide à un patient, comprenant :
un réservoir (2) pour un fluide ;
une première pompe (10) comprenant des premier et deuxième orifices (14, 15) de pompe ; et
un ensemble (22) de vanne comprenant des premier, deuxième et troisième orifices (28, 29, 30) de vanne ; dans lequel :
le premier orifice (28) de vanne est en communication fluidique avec le réservoir (2) ;
le deuxième orifice (29) de vanne est en communication fluidique avec le premier orifice (14) de pompe ;
le troisième orifice (30) de vanne est une sortie pour administrer un fluide à un patient ;
le deuxième orifice (15) de pompe est en communication fluidique avec le réservoir (2) ; et **caractérisé en ce que**
la première pompe (10) est réversible de sorte que :
dans un mode de recirculation, l'ensemble (22) de vanne est configuré pour permettre l'écoulement de fluide du premier orifice (28) de vanne au deuxième orifice (29) de vanne mais pas au troisième orifice (30) de vanne, la pompe (10) étant actionnée dans une direction pour aspirer le fluide du réservoir (2) au premier orifice (14) de pompe en passant par l'ensemble (22) de vanne et pour faire recirculer le fluide du deuxième orifice (15) de pompe au réservoir (2) ; et
dans un mode d'administration de fluide, l'ensemble (22) de vanne est configuré pour permettre l'écoulement de fluide du deuxième orifice (29) de vanne au troisième orifice (30) de vanne mais pas au premier orifice (28) de vanne, la pompe (10) étant actionnée dans une direction opposée pour aspirer le fluide du réservoir (2) au deuxième orifice (15) de pompe et pour administrer le fluide du premier orifice (14) de pompe au troisième orifice (30) de vanne en passant par l'ensemble (22) de vanne.

2. Appareil d'administration de fluide selon la revendication 1, dans lequel l'ensemble (22) de vanne est configuré de sorte que les seuls écoulements de fluide permis entre les premier, deuxième et troisième orifices (28, 29, 30) de vanne sont du premier orifice (28) de vanne au deuxième orifice (29) de vanne et du deuxième orifice (29) de vanne au troisième orifice (30) de vanne.

3. Appareil d'administration de fluide selon la revendication 1, dans lequel l'ensemble (22) de vanne comprend une première vanne (38) permettant un écoulement unidirectionnel du premier orifice (28) de vanne au deuxième orifice (29) de vanne ; une deuxième vanne (40) permettant un écoulement unidirectionnel du deuxième orifice (29) de vanne au troisième orifice (30) de vanne ; et aucune communication directe entre les premier et troisième orifices (28, 30) de vanne.

4. Appareil d'administration de fluide selon une quelconque revendication précédente, dans lequel la première pompe (10) est une pompe péristaltique.

5. Appareil d'administration de fluide selon la revendication 4, dans lequel la pompe péristaltique (10) comprend des rouleaux qui compriment un tube (18) élastique contre une surface (8) arquée, la surface arquée (8) étant formée d'une seule pièce avec une paroi du réservoir (2).

6. Appareil d'administration de fluide selon une quelconque revendication précédente, dans lequel les premier et deuxième orifices (14, 15) de pompe s'ouvrent dans un intérieur du réservoir (2), comprenant en outre un premier tube (34) de transfert qui traverse le réservoir (2) pour assurer la communication fluidique entre le deuxième orifice (29) de vanne et le premier orifice (14) de pompe.

7. Appareil d'administration de fluide selon une quelconque revendication précédente, comprenant en outre :
un chauffage (20) pour chauffer le fluide dans le réservoir (2) ; et
un capteur (21) de température agencé pour surveiller la température du fluide dans l'appareil et pour fournir un signal de température qui peut être utilisé pour commander le chauffage (20).

8. Appareil d'administration de fluide selon l'une quelconque des revendications 1 à 7, comprenant en outre un orifice (56) de retour par gravité en communication fluidique avec un filtre (66), moyennant quoi le fluide introduit par l'orifice (56) de retour par gravité doit traverser le filtre (66) pour s'écouler dans le réservoir (2).

9. Appareil d'administration de fluide selon l'une quelconque des revendications 1 à 7, comprenant en outre :
un orifice (48) de retour d'aspiration ;
un filtre (66) ;
un orifice (58) d'entrée de filtre en communication fluidique avec le filtre (66) ; et
une deuxième pompe (12) comprenant des troisième et quatrième orifices (16, 17) de pompe ;
dans lequel :
l'orifice (48) de retour d'aspiration est en communication fluidique avec le troisième orifice (16) de pompe ;
le quatrième orifice (17) de pompe est en communication fluidique avec l'orifice (58) d'entrée de filtre ; et
dans le mode d'administration de fluide, la deuxième pompe (12) est actionnée pour aspirer du fluide de l'orifice (48) de retour d'aspiration au troisième orifice (16) de pompe et pour administrer un fluide du quatrième orifice (17) de pompe à l'orifice (58) d'entrée de filtre, moyennant quoi le fluide introduit par l'orifice (58) d'entrée de filtre doit traverser le filtre (66) pour s'écouler dans le réservoir (2).

10. Appareil d'administration de fluide selon la revendication 9, dans lequel la deuxième pompe (12) est une pompe péristaltique.

11. Appareil d'administration de fluide selon la revendication 10, dans lequel la pompe (12) péristaltique comprend des rouleaux qui compriment un tube élastique contre une surface arquée, la surface arquée étant formée d'une seule pièce avec une paroi du réservoir (2).

12. Appareil d'administration de fluide selon l'une quelconque des revendications 9 à 11, dans lequel les troisième et quatrième orifices (16, 17) de pompe sont situés dans le réservoir (2), comprenant en outre un deuxième tube (54) de transfert qui traverse le réservoir (2) pour assurer la communication fluidique entre l'orifice (48) de retour d'aspiration et le troisième orifice (16) de pompe.

13. Appareil d'administration de fluide selon l'une quelconque des revendications 9 à 12, comprenant en outre un orifice (56) de retour par gravité en communication fluidique directe avec le filtre (66), moyennant quoi le fluide introduit par l'orifice (56) de retour par gravité doit traverser le filtre (66) pour s'écouler dans le réservoir (2).

14. Appareil d'administration de fluide selon une quelconque revendication précédente, comprenant en outre un raccord (70) qui comprend :
un trou traversant s'étendant d'une première extrémité du raccord (70) à une deuxième extrémité du raccord (70) ;
un premier accouplement (86) à la première extrémité pour fixer le raccord (70) à un tube (36) flexible ; et
un deuxième accouplement à la deuxième extrémité pour fixer de manière non amovible le raccord (70) au troisième orifice (30) de vanne.

15. Appareil d'administration de fluide selon la revendication 14, dans lequel le deuxième accouplement est un accouplement à encliquetage, qui comprend une pluralité de bras (82) élastiques longitudinaux qui traversent le troisième orifice (30) de vanne, chaque bras se terminant par un tenon (84) qui bondit radialement vers l'extérieur pour être en prise avec une surface d'extrémité de l'orifice (30) et empêcher le retrait du raccord (70) à travers l'orifice (30).
